# EUROPEAN PATENT APPLICATION

(11) **EP 4 706 619 A1**
(43) Date of publication of application: **11.03.2026**
(21) Application number: 25199946.2
(22) Date of filing: 03.09.2025
(51) Int. Cl.: A61G 7/018, A61G 7/05

(54) **PATIENT SUPPORT APPARATUS HAVING ENDBOARD WITH INTEGRATED ELECTRICAL SYSTEM INPUT**

(30) Priority: 03.09.2024 US 202463689933 P; 24.02.2025 US 202563762167 P
(71) Applicant: Hill-Rom Services, Inc., Batesville, IN 47006-9167 (US)
(72) Inventor: MOUFLIER, Maxime Antoine, Batesville, 47006-9167 (US); MOREL, Thomas, Batesville, 47006-9167 (US); DONVAL, Jacques Antoine, Batesville, 47006-9167 (US)
(74) Representative: Reddie & Grose LLP

(57) **Abstract**

A patient support apparatus includes a frame and an electrical system coupled to the frame and operable to operate an electrical function of the patient support apparatus. An endboard is removably coupled to the frame by a coupling mechanism that provides an electrical connection to a mating coupling mechanism of the frame. The endboard has a user input in electrical communication with the coupling mechanism so that an input received from a user at the user input is communicated electrically to the electrical system via the electrical connection to operate an electrical function of the patient support apparatus.

## Description

The present disclosure relates to patient support apparatuses and, in particular, to patient support apparatuses having user inputs to control powered propulsion systems that drive patient support apparatus along an underlying floor.

It is known that patient support apparatuses, for example, patient beds and electrically powered stretchers, include user inputs for electronically controlling one or more electrical components of the patient support apparatus. Some patient beds or stretchers optionally may include a powered propulsion system having a motorized or powered wheel. Accordingly, such patient support apparatuses also include user inputs for activating the powered wheel or, more specifically, for activating a drive device such as a motor that is configured to rotate the wheel to assist a caregiver when moving the patient support apparatus. In some prior art embodiments of this type, the user inputs include controls that are incorporated into push handles of the patient support apparatus.

Some patient support apparatuses have endboards that include control panels or graphical user interfaces that provide user inputs for electronically controlling components of the patient support apparatus, but not the powered propulsion system. This is because it is desirable for the endboards to be removable to enhance patient access by caregivers, but yet control of the powered propulsion system is still desired with the endboard removed. Hence the use of push handles for this purpose. However, the push handles also may serve as an impediment that blocks access to the patient. As a result, the push handles having controls for powered propulsion system are typically not removable from the bedframe but are foldable downwardly to a storage position which adds to the cost and complexity of the push handles. Also, for endboards having control panels or graphical user interfaces, an electrical connector is typically provided separately from the mechanical structure, such as posts or sockets, which is used to removably coupled the endboards with the associated bed frames. Thus, there is an ongoing need for improvements in controls for powered propulsion systems of patient support apparatuses.

The present disclosure includes one or more of the following features alone or in any combination,.

According to a first aspect of the disclosed embodiments, a patient support apparatus includes a frame. A propulsion system is coupled to the frame and is operable to propel the patient support apparatus along a floor. An endboard is removably coupled to the frame by a coupling mechanism that provides an electrical connection to a mating coupling mechanism of the frame. The endboard has a user input in electrical communication with the coupling mechanism so that an input received from a user at the user input is communicated electrically to the propulsion system via the electrical connection to operate the propulsion system to propel the patient support apparatus along the floor.

In some embodiments of the first aspect, the propulsion system may include a powered wheel configured to move the patient support apparatus. The powered wheel may be activated when the input is received from the user at the user input. The propulsion system may include an electrical brake configured to prevent movement of the patient support apparatus. The electrical brake may be activated when the user input is released by the user. The user input may include a plurality of activation states. The powered wheel may move the patient support apparatus in a first direction, when the user input is in a first activation state. The powered wheel may move the patient support apparatus in a second direction, when the user input is in a second activation state. The electrical brake may be released and the powered wheel may be placed in idle, when the user input is in an idle activation state. The powered wheel may begin moving the patient support apparatus, when the user input is in a movement activation state.

Optionally, in the first aspect, the coupling mechanism may include a post configured to be inserted into the mating coupling mechanism. The mating coupling mechanism may include a post configured to be inserted into the coupling mechanism. The endboard may be a footboard. The endboard may be a headboard. A locking mechanism may be configured to secure the endboard to the frame. The locking mechanism may rotate to provide the electrical connection between the coupling mechanism and the mating coupling mechanism. The locking mechanism may include a spring blade to provide the electrical connection between the coupling mechanism and the mating coupling mechanism. The user input may be a first user input. The endboard may include a second user input. The first user input may be positioned on a first side of the endboard. The second user input may be positioned on a second side of the endboard. Both the first user input and the second user input may be required to be engaged to control the propulsion system of the patient support apparatus.

It may be desired, in the first aspect, that a light source illuminates the user input. The light source may illuminate the user input in a color that indicates that the propulsion system is capable of activation. The light source may illuminate the user input in a color that indicates that the propulsion system requires charging. The light source may illuminate the user input in a color that indicates that the propulsion system is charging. A brightness of the light source may indicate a charging level of the propulsion system.

According to a second aspect of the disclosed embodiments, a patient support apparatus includes a frame. An endboard is configured to couple to the frame. The endboard includes a user input configured to control a propulsion system of the patient support apparatus. A post extends from the endboard. A channel is formed in a sleeve positioned in the frame. The sleeve has a notch formed therein. The endboard is coupled to the frame by inserting the post into the channel in the sleeve. An electrical connection is aligned with the sleeve. The electrical connection is configured to extend through the notch formed in the sleeve to engage the post when the endboard is coupled to the frame so that an input received from a user at the user input is communicated electrically to the propulsion system to operate the propulsion system to propel the patient support apparatus along the floor.

In some embodiments of the second aspect, the propulsion system may include a powered wheel configured to move the patient support apparatus. The powered wheel may be activated when the input is received from the user at the user input. The propulsion system may include an electrical brake configured to prevent movement of the patient support apparatus. The electrical brake may be activated when the user input is released by the user. The user input may include a plurality of activation states. The powered wheel may move the patient support apparatus in a forward direction, when the user input is in a first activation state. The powered wheel may move the patient support apparatus in a backward direction, when the user input is in a second activation state. The electrical brake may be released and the powered wheel may be placed in idle, when the user input is in an idle activation state. The powered wheel may begin moving the patient support apparatus, when the user input is in a movement activation state.

Optionally, in the second aspect, the endboard may be a footboard. The endboard may be a headboard. A locking mechanism may be configured to secure the endboard to the frame. The locking mechanism may rotate the sleeve so that the electrical connection extends though the notch. The user input may be a first user input. The patient support apparatus may include a second user input. The first user input may be positioned on a first side of the endboard. The second user input may be positioned on a second side of the endboard. Both the first user input and the second user input may be required to be engaged to control the propulsion system.

It may be contemplated, in the second aspect, that a light source illuminates the user input. The light source may illuminate the user input in a color that indicates that the propulsion system is capable of activation. The light source may illuminate the user input in a color that indicates that the propulsion system requires charging. The light source may illuminate the user input in a color that indicates that the propulsion system is charging. A brightness of the light source may indicate a charging level of the propulsion system.

According to a third aspect of the disclosed embodiments, a patient support apparatus includes a frame. An endboard is configured to couple to the frame. The endboard has a user input configured to control a propulsion system of the patient support apparatus. A channel is formed in a sleeve positioned in the endboard. The sleeve has a notch formed therein. A post extends from the frame. The endboard is coupled to the frame by inserting the post into the channel in the sleeve. An electrical connection is aligned with the sleeve. The electrical connection is configured to extend through the notch formed in the sleeve to engage the post when the endboard is coupled to the frame so that an input received from a user at the user input is communicated electrically to the propulsion system to operate the propulsion system to propel the patient support apparatus along the floor.

According to a fourth aspect of the disclosed embodiment, a patient support apparatus includes a base frame having a longitudinal axis extending between a head end and a foot end. A plurality of casters is coupled to the base frame. The plurality of casters includes at least one powered wheel. An articulating deck is positioned above the base frame and configured to carry a patient support surface. A pair of lift arms couple the base frame to the articulating deck. The pair of lift arms are laterally spaced in a direction substantially perpendicular to the longitudinal axis to define a space therebetween. The pair of lift arms are configured to move the articulating deck relative to the base frame. An electronic unit is electrically coupled to the at least one powered wheel. The electronic unit is carried by the pair of lift arms and situated within the space defined between the pair of lift arms.

In some embodiments of the fourth aspect, the pair of lift arms may be pivoted in unison to raise and lower a portion of the articulating deck relative to the base frame. The electronic unit may include a battery for powering the at least one powered wheel and/or a motor controller to signal operation of the at least one powered wheel. Lower ends of the pair of lift arms may move longitudinally along the base frame as the articulating deck is moved by the pair of lift arms. The electronic unit may be configured to move with the pair of lift arms when the articulating deck is moved relative to the base frame.

Optionally, in the fourth aspect, the at least one powered wheel may be activated with a user input that is engaged manually by a user. Engagement of the user input by the user may be ineffective to activate the at least one powered wheel until the at least one powered wheel is locked into a steering position. In the steering position, the at least one powered wheel may be positioned to rotate about a wheel axis that is substantially perpendicular to the longitudinal axis.

It may be contemplated in the fourth aspect, that a lift actuator may be operable to move the pair of lift arms. The electronic unit may also be electrically coupled to the actuator. At least one deck actuator may be operable to move at least one deck section of the articulating deck. The electronic unit may also be electrically coupled to the deck actuator.

It may be desired, in the fourth aspect, that an electrical connection between the electronic unit and the at least one powered wheel may extend through the base frame. The electronic unit may be accessible when the articulating deck is in a raised position to allow at least one of mounting or changing of the electronic unit. The electronic unit may be accessible with a patient positioned on the patient support surface.

In some embodiments of the fourth aspect, the pair of lift arms may include at least one of a head end pair of lift arms or a foot end pair of lift arms. The electronic unit may include a first electronic unit coupled to the head end pair of lift arms and a second electronic unit coupled to the foot end pair of lift arms. The first and second electronic units may each include a battery. The first and second electronic units may each include a motor controller. The first and second electronic units may each include a battery and a motor controller. A first lift actuator may be operable to move the head end pair of lift arms and a second lift actuator may be operable to move the foot end pair of lift arms. The first electronic unit may be electrically coupled to the first lift actuator and the second electronic unit may be electrically coupled to the second lift actuator.

According to a fifth aspect of the disclosed embodiments, a patient support apparatus includes a base frame having at least one powered wheel coupled thereto. An articulating deck is positioned above the base frame and configured to carry a patient support surface. A lift assembly is couples the base frame to the articulating deck and is configured to move the articulating deck relative to the base frame. An electronic unit is electrically coupled to the at least one powered wheel. The electronic unit is carried by and moves in conjunction with the lift assembly.

In some embodiments of the fifth aspect, the lift assembly may raise and lower a portion of the articulating deck relative to the base frame. The electronic unit may include a battery for powering the at least one powered wheel and/or a motor controller to signal operation of the at least one powered wheel. A lower end of the lift assembly may move longitudinally along the base frame as the articulating deck is moved by the lift assembly.

Optionally, in the fifth aspect, the at least one powered wheel may be activated with a user input that is engaged manually by a user. Engagement of the user input by the user may be ineffective to activate the at least one powered wheel until the at least one powered wheel is locked into a steering position. In the steering position, the at least one powered wheel may be positioned to rotate about a wheel axis that is substantially perpendicular to the longitudinal axis.

It may be contemplated, in the fifth aspect, that a lift actuator may be operable to move the lift assembly. The electronic unit may also be electrically coupled to the actuator. At least one deck actuator may be operable to move at least one deck section of the articulating deck. The electronic unit may also be electrically coupled to the deck actuator.

It may be desired, in the fifth aspect, that an electrical connection between the electronic unit and the at least one powered wheel may extend through the base frame. The electronic unit may be accessible when the articulating deck is in a raised position to allow at least one of mounting or changing of the electronic unit. The electronic unit may be accessible with a patient positioned on the patient support surface.

In some embodiments of the fifth aspect, the lift assembly may include at least one of a head end lift assembly or a foot end lift assembly. The electronic unit may include a first electronic unit coupled to the head end lift assembly and a second electronic unit coupled to the foot end lift assembly. The first and second electronic units may each include a battery. The first and second electronic units may each include a motor controller. The first and second electronic units may each include a battery and a motor controller. A first lift actuator may be operable to move the head end lift assembly and a second lift actuator may be operable to move the foot end lift assembly. The first electronic unit may be electrically coupled to the first lift actuator and the second electronic unit may be electrically coupled to the second lift actuator.

According to a sixth aspect of the disclosed embodiments, a patient support apparatus includes a base frame having at least one powered wheel coupled thereto. The at least one powered wheel is controlled by a control circuit. An articulating deck is positioned above the base frame and is configured to carry a patient support surface. A plurality of linkages is configured to move the articulating deck relative to the base frame. The plurality of linkages includes a lift arm configured to raise and lower the articulating deck relative to the base frame. An electronic unit electrically couples to each of the at least one powered wheel, the linkages, and the control circuit. The electronic unit is carried by the lift arm.

In some embodiments of the sixth aspect, the lift arm may raise and lower a portion of the articulating deck relative to the base frame. The electronic unit may include a battery for powering the at least one powered wheel and/or a motor controller to signal operation of the at least one powered wheel. A lower end of the lift arm may move longitudinally along the base frame as the articulating deck is moved by the lift arm. The electronic unit may be configured to move with the lift arm when the articulating deck is moved relative to the base frame.

Optionally, in the sixth aspect, the at least one powered wheel may be activated with a user input that is engaged manually by a user. Engagement of the user input by the user may be ineffective to activate the at least one powered wheel until the at least one powered wheel is locked into a steering position. In the steering position, the at least one powered wheel may be positioned to rotate about a wheel axis that is substantially perpendicular to the longitudinal axis.

It may be desired, in the sixth aspect, that an electrical connection between the electronic unit and the at least one powered wheel may extend through the base frame. The electronic unit may be accessible when the articulating deck is in a raised position to allow at least one of mounting or changing of the electronic unit. The electronic unit may be accessible with a patient positioned on the patient support surface.

It may be contemplated, in the sixth aspect, that the lift arm may include at least one of a head end lift arm or a foot end lift arm. The electronic unit may include a first electronic unit coupled to the head end lift arm and a second electronic unit coupled to the foot end lift arm. The first and second electronic units may each include a battery. The first and second electronic units may each include a motor controller. The first and second electronic units may each include a battery and a motor controller. A first lift actuator may be operable to move the head end lift arm and a second lift actuator may be operable to move the foot end lift arm. The first electronic unit may be electrically coupled to the first lift actuator and the second electronic unit may be electrically coupled to the second lift actuator.

According to a seventh aspect of the disclosed embodiments, a patient support apparatus includes a frame and an electrical system coupled to the frame and operable to operate an electrical function of the patient support apparatus. An endboard is removably coupled to the frame by a coupling mechanism that provides an electrical connection to a mating coupling mechanism of the frame. The endboard has a user input in electrical communication with the coupling mechanism so that an input received from a user at the user input is communicated electrically to the electrical system via the electrical connection to operate an electrical function of the patient support apparatus. A locking mechanism is configured to secure the endboard to the frame. The locking mechanism is moveable between a first position and a second position to provide the electrical connection between the coupling mechanism and the mating coupling mechanism. In some embodiments of the seventh aspect, an electronic unit is electrically coupled to a powered wheel. The electronic unit is carried by a pair of lift arms and situated within a space defined between the pair of lift arms

The invention will now be further described by way of example with reference to the accompanying drawings, in which:
FIG. 1 is a perspective view of a patient support apparatus showing an endboard having user inputs to activate a motorized propulsion system of the patient support apparatus;
FIG. 2 is a perspective view of the endboard of the patient support apparatus removed from a frame of the patient support apparatus;
FIG. 3 is a perspective view of the endboard of the patient support apparatus showing an electrical connection between user inputs on the endboard and a coupling mechanism of the endboard;
FIG. 4 is perspective view of a locking mechanism carried by the frame of the patient support apparatus and configured to lock the endboard to the frame;
FIG. 5 is a front view of the endboard coupled to the frame and a pair of locking mechanisms is respective disengaged or released positions;
FIG. 6 is a cross-sectional view, taken along line 6-6 of FIG. 5, of one of the coupling mechanisms of the endboard coupled to the frame and the associated locking mechanism in the disengaged position;
FIG. 7 is a front view, similar to FIG. 5, showing the endboard coupled to the frame and the pair of locking mechanisms is respective engaged or locked positions;
FIG. 8 is a cross-sectional view, taken along line 8-8 of FIG. 7, of one of the coupling mechanisms of the endboard coupled to the frame and the associated locking mechanism in the engaged position;
FIG. 9 is a side view of the patient support apparatus shown in FIG. 1;
FIG. 10 is a top view of the powered wheel shown in FIG. 1 positioned in a rotated position;
FIG. 11 is a top view of the powered wheel shown in FIG. 1 positioned in a steering position;
FIG. 12 is a side view of the patient support apparatus shown in FIG. 1 with a control circuit and a plurality of actuators shown in dashed lines;
FIG. 13 is a perspective view of the lift assembly positioned at the foot end of the patient support apparatus shown in FIG. 1;
FIG. 14 is a front view of the lift assembly positioned at the foot end of the patient support apparatus shown in FIG. 1;
FIG. 15 is a top perspective view of a first electronic unit coupled between the lift arms of the lift assembly shown in FIGs. 13-14, wherein a cover panel is illustrated as transparent to show the components positioned under the cover panel; and
FIG. 16 is a bottom view of another first electronic unit coupled between the lift arms of the lift assembly shown in FIGs. 13-14.

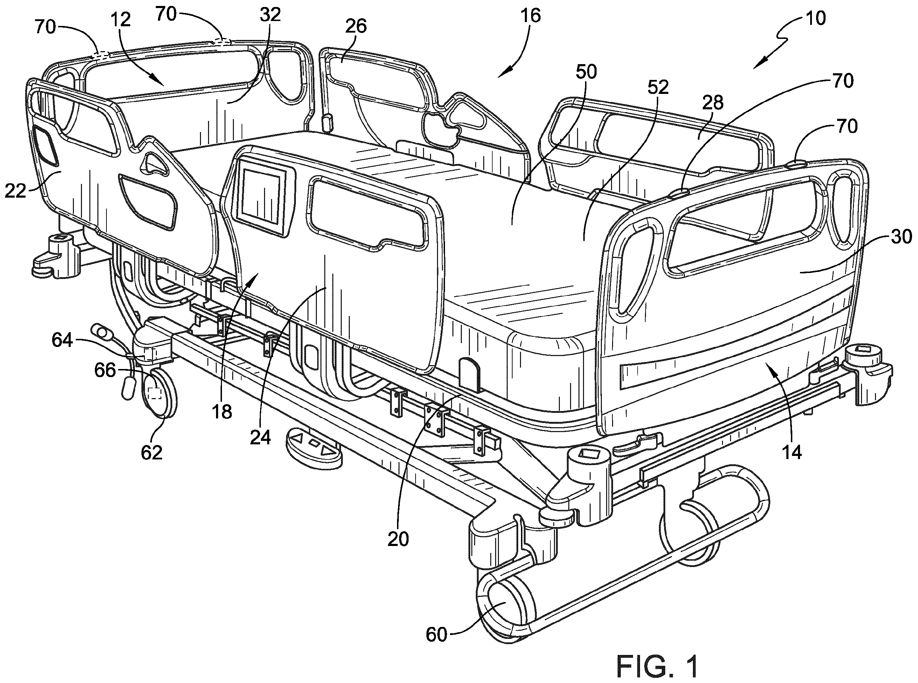

Referring to Fig. 1, a patient support apparatus 10 in accordance with the present disclosure is embodied as a patient bed. The patient support apparatus 10 includes a head end 12, a foot end 14, a first side 16, and second side 18, with the first and second siders 16, 18 extending between the head end 12 and the foot end 14. The patient support apparatus 10 includes a frame 20. Right siderails 22, 24 and left siderails 26, 28 are coupled to and extend generally upwardly from an upper frame portion of the frame 20, but are each movable to respective lowered positions. As used herein, "left" and "right" are designated from the perspective of a patient lying supine on the patient support apparatus 10 with their head adjacent the head end 12. An endboard 30, embodied as a footboard, extends from the frame 20 at the foot end 14 of the patient support apparatus. An endboard 32, embodied as a headboard, extends from the frame 20 at the head end 12 of the patient support apparatus. A mattress 50 is carried by the frame 20 and provides a sleeping surface or support surface 52 configured to receive a patient thereon.

While the illustrative patient support apparatus 10 is a patient bed, the present disclosure is also applicable to other types of patient support apparatuses such as stretchers, mobile chairs, surgical tables, and the like having endboards (e.g., footboard 30 and/or headboard 32) or structures akin to endboards that are used to push, pull, or otherwise maneuver the corresponding patient support apparatus along a floor. As shown in Fig. 1, when the siderails 22, 24, 26, 28 are in their raised positions and the endboards 30, 32 are attached to the frame 20, the siderails 22, 24, 26, 28 and endboards 30, 32 extend upwardly beyond an upper surface of the mattress 50 to serve as barriers that inhibit a patient from exiting the patient bed 10. A plurality of casters 60 are coupled to a base frame portion of frame 20 and are configured to roll and swivel when a caregiver moves the patient support apparatus 10.

In the illustrative embodiment, an electrical system is coupled to the frame 20 and is operable to operate an electrical function of the patient support apparatus. For example, in the illustrative embodiment, one of the casters 60 includes a propulsion system having a powered wheel 62 configured to drive the patient support apparatus 10 by rotating the powered wheel 62 and an electrical brake 66 configured to prevent movement of the powered wheel 62 and the patient support apparatus 10. Optionally, others of casters 60 are similarly powered with powered wheels 62 in other embodiments. In some embodiments, each of the powered wheels 62 is available from TENTE International GmbH of Cologne, Germany and a control system for driving the powered wheel is available from Linak A/S of Guderup, Denmark. Accordingly, the powered wheel 62 includes a motor 64 that is configured to drive the powered wheel 62 when activated and that is configured to brake the powered wheel via electromotive force (EMF) when deactivated. Because the caster 60 is freely swivelable about a generally vertical axis, the motor 64 is activated in only a single angular direction in the illustrative embodiment, and the user dictates the direction of propulsion via manual forces on the patient support apparatus 10 to swivel the caster 60 into the desired trailing orientation for propulsion. It will be appreciated that, in some embodiments, the electrical system is configured to operate other systems of the patient support apparatus 10, for example, lift motors, deck motors, a blower configured to inflate an air blader of the mattress 50, a blower configured to supply air to a microclimate management system, or the like.

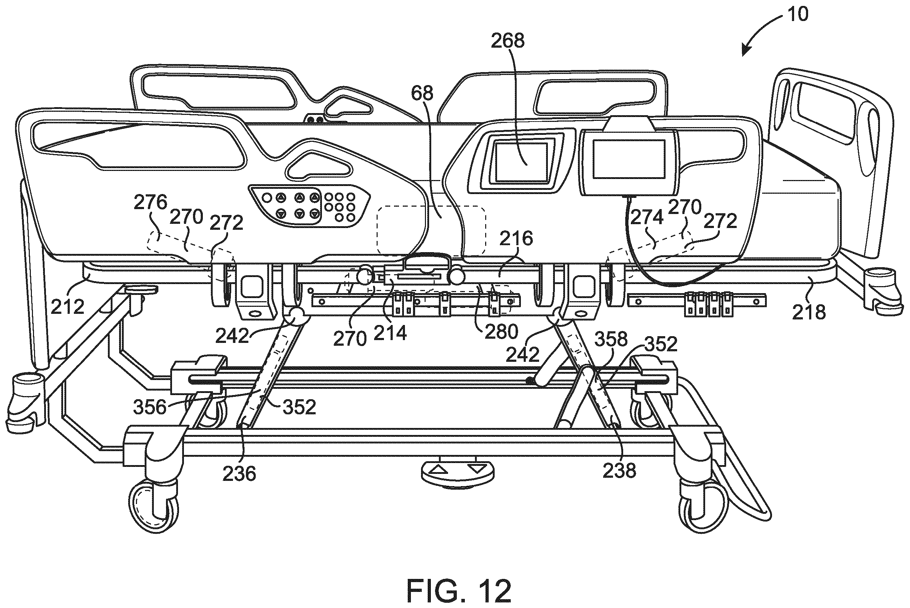

In some embodiments, the powered wheel 62 and an electrical brake 66, if provided separately from the motor 64, are controlled with signals from a control circuitry 68 (shown in FIG. 12) of the patient support apparatus 10. In some embodiments, the powered wheel 62 will only provide assistance to the user or caregiver moving the patient support apparatus 10, if the powered wheel 62 senses the patient support apparatus 10 is moving. That is, the powered wheel 62 can be powered, but if the caregiver does not provide an initial moving force to the patient support apparatus 10, the powered wheel 62 will not provide assistance. To allow for initial rotational motion of the powered wheel 62, the electric brake is released.

The endboard 30 includes user inputs 70 that receive inputs from a user that are communicated electrically to the electrical system to operate an electrical function of the patient support apparatus. In an exemplary embodiment, the user inputs 70 are configured to control a component, particularly the powered wheel 62 and/or the electrical brake 66, of the patient support apparatus 10. In other embodiments, the user inputs 70 are configured to control other components of the patient support apparatus 10, for example, lift motors, deck motors, a blower configured to inflate an air blader of the mattress 50, a blower configured to supply air to a microclimate management system, or the like. It will be appreciated that although FIG. 1 illustrates a pair of user inputs 70, the patient support apparatus 10 includes only one user input 70 in other contemplated embodiments. In some embodiments, the user inputs 70 include buttons, levers, switches, or any suitable user input. Additionally, although the user inputs 70 are illustrated on the endboard 30, it will be appreciated that the user inputs are located on the endboard 32, in some embodiments, as shown in FIG. 1 (in phantom) in lieu of, or in addition to, the buttons 70 on the endboard 30. For example, in Europe, it is common to push patient support apparatuses from the foot end 14 and so, having buttons 70 on the footboard 30 is the preferred arrangement. In contrast, in the United States, it is common to push patient support apparatus from the head end 12 and so, having buttons 70 on the headboard 32 is the preferred arrangement. The present disclosure contemplates that, in some embodiments, endboards 30, 32 can each be configured to selectively mount to the head end 12 or the foot end 14 of the patient support apparatus 10. That is, whether the endboard is considered to be a footboard 30 or a headboard 32 is dictated by which end 12, 14 of the frame 20 the particular endboard is mounted.

In the illustrative example, the powered wheel 62 is activated when both of the user inputs 70 are engaged by a user and initial rolling motion of the powered wheel is sensed. Furthermore, the electrical brake 66 is activated when either of the user inputs 70 is released by the user. Requiring both inputs 70 to be engaged before powered propulsion is possible serves as a safety feature to prevent inadvertent propulsion of powered wheel 62, such as if only one of inputs 70 is engaged. Basically, inputs 70 are fashioned as switches in series that are normally in an opened state and so, both inputs 70 need to be engaged so that the series switches are in a closed state permitting current flow in the power propulsion input circuit. In some embodiments, the user inputs 70 include a plurality of activation states. That is, the user inputs 70 can be switched to different positions that correspond to different actions actuated by the electrical components. In one embodiment, the powered wheel 62 moves the patient support apparatus 10 in a forward direction, when the user inputs 70 are in a forward activation state, and the powered wheel 62 moves the patient support apparatus 10 in a backward direction, when the user inputs 70 are in a backward activation state. Such an arrangement would be desirable, for example, in embodiments having non-castered (e.g. non-swivelable) drive wheels. In some embodiments, the electrical brake 66 is released and the powered wheel 62 is placed in idle, when the user inputs 70 are in an idle activation state, and wherein the powered wheel 62 begins moving the patient support apparatus 10, when user inputs 70 are in a movement activation state.

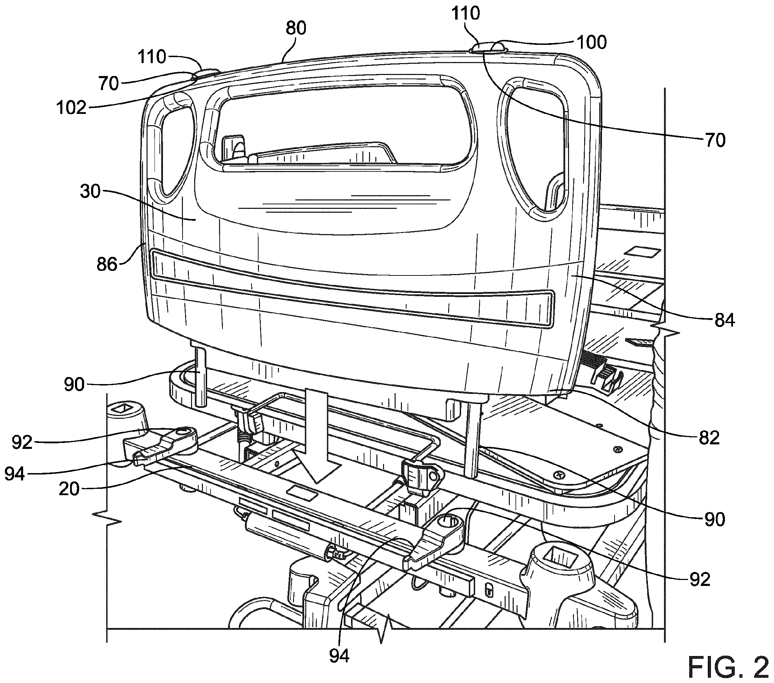

Referring now to FIG. 2, the endboard 30 is shown removed from the frame 20 of the patient support apparatus 10. The endboard 30 includes a top 80 and an opposite bottom 82. A left side 84 and a right side 86 extend between the top 80 and the bottom 82. A pair of coupling mechanisms 90 extend from the bottom 82 of the endboard 30. In the exemplary embodiment, each coupling mechanism 90 is configured as a post. In some embodiments, the endboard 30 includes any number of coupling mechanisms 90. Mating coupling mechanisms 92 are provided in the frame 20. In the exemplary embodiment, each mating coupling mechanism 92 is configured as a socket configured to receive a corresponding coupling mechanism 90 configured as a post. The frame 20 includes a number of mating coupling mechanisms 92 that corresponds to the number of coupling mechanisms 90 of the endboard 30. The endboard 30 is configured to removably couple to the frame 20 by inserting the coupling mechanisms 90 configured as posts on the endboard 30 into the mating coupling mechanisms 92 configured as sockets in the frame 20. Endboard 32 also has coupling mechanisms 90 and the head end 12 of frame 20 includes coupling mechanisms 92 like those described herein in connection with endboard 30.

A locking mechanism 94 is positioned on the frame 20 adjacent each mating coupling mechanism 92. In the exemplary embodiment, the frame 20 includes one locking mechanism 94 for each mating coupling mechanism 92. In some embodiments, the frame 20 only includes one locking mechanism 94. The locking mechanisms 94 are configured has levers or handles that rotate between an engaged position and a disengaged position. In the engaged position, the locking mechanisms 94 engage the respective coupling mechanism 90 when the coupling mechanism 90 is inserted into the mating coupling mechanism 92.

In some embodiments, the coupling mechanisms 90 are configured as posts and extend upwardly from the frame 20. In such embodiments, the mating coupling mechanisms 92 are configured as sockets that are formed in the endboard 30 so that the endboard 30 is removably coupled to the frame 20 by sliding the mating coupling mechanisms 92 configured as sockets in the endboard 30 over the coupling mechanisms 90 configured as posts on the frame 20. Additionally, in such embodiments, the locking mechanisms 94 are positioned on the endboard 30 adjacent the mating coupling mechanisms 92 configured as sockets in the endboard 30.

In the illustrative example, the user inputs 70 include a first user input embodied as a button 100 and a second user input embodied as a button 102. In an exemplary embodiment, the user inputs 70 are positioned on the top 80 of the endboard 30. In some embodiments, the user input 100 is positioned on the left side 84 of the top 80 of the endboard 30 and the user input 102 is positioned on the right side 86 of the top 80 of the endboard 30. In an exemplary embodiment, both the user input 100 and the user input 102 are required to be engaged by a user or caregiver to activate the propulsion system of the patient support apparatus 10 as mentioned above. It will be appreciated that the endboard 30 may include only one user input 70 at any position, in some embodiments as also mentioned above.

In some embodiments, a light source 110 illuminates each user input 70. In such an embodiment, the light source 110 illuminates the user inputs 70 in a color that indicates that the powered wheel 62 of the patient support apparatus is capable of activation. That is, if the electrical brake 66 and/or the powered wheel 62 are unlocked and capable of being activated, the lights source 110 illuminates the user inputs 70 in a predefined color. In some embodiments, the light source 110 illuminates the user inputs 70 in a predefined color that indicates that the component (i.e. the electrical brake 66 and/or the powered wheel 62) of the patient support apparatus 10 requires charging. In some embodiments, the light source 110 illuminates the user inputs 70 in a predefined color that indicates that the component (i.e. the electrical brake 66 and/or the powered wheel 62) of the patient support apparatus 10 is currently charging. In some embodiments, a brightness of the light source 110 indicates a charging level of the component (i.e. the electrical brake 66 and/or the powered wheel 62) of the patient support apparatus 10. For example, if the component is charged below a predetermined percentage, the light source 110 is dim, but of the component is charged above the predetermined percentage, the light source 110 is bright. In some embodiments, the brightness of the light source 110 increases commensurate with a percentage of charge of the component.

The light source 110 in the illustrative example is fashioned as a bezel or oblong ring that surrounds the respective user input 70. The light bezel is fixed relative to the top 80 of the endboard 30 and the buttons 100, 102 move vertically downwardly within the light bezel when pressed downwardly by a user to activate the propulsion system. The buttons 100, 102 are spring biased to move upwardly relative to the light bezel when the user releases the buttons 100, 102 to deactivate the propulsion system. In other embodiments, buttons 100, 102 are translucent and are, themselves, illuminated in lieu of having illuminable light bezels.

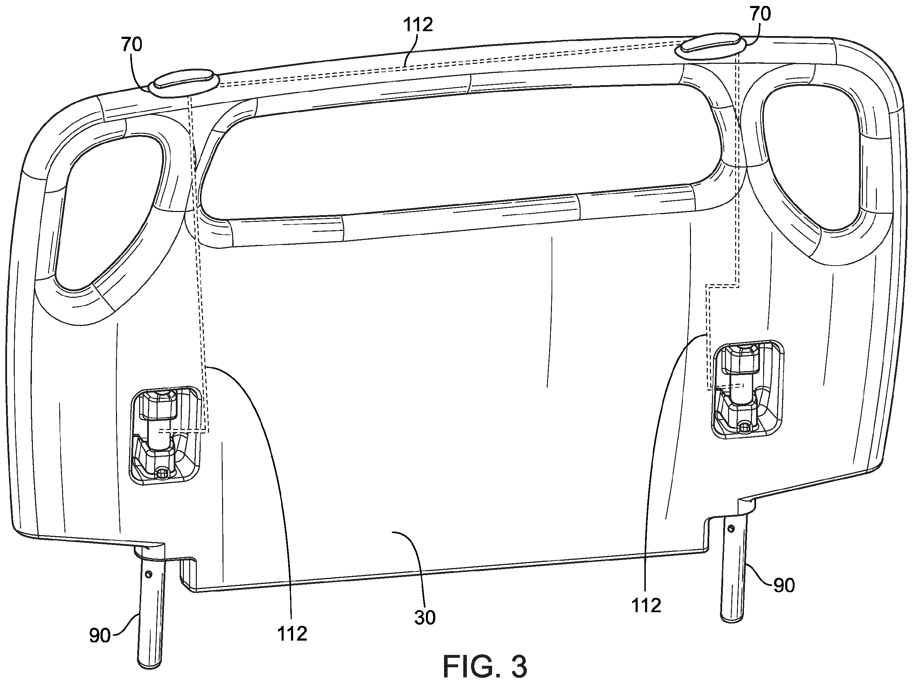

Referring now to FIG. 3 an electrical connection 112 (e.g., one or more wires or other electrical conductors) extends between the user inputs 70 of the endboard 30 and from each of the user inputs 70 to the respective coupling mechanism 90 of the endboard 30 to wire the user inputs 70 in series to the coupling mechanisms 90. The electrical conductors of connection 112 are routed through an interior region of endboard 30 so are not visible outside of endboard 30. The generally rectangular pockets within which upper ends of posts 90 are accessible, and in which a small portion of respective conductors 112 can be seen, are closed off by cosmetic covers (not shown) in use. In an exemplary embodiment, the coupling mechanisms 90 are conductive and configured to allow electrical current to flow through the user inputs 70 when engaged. That is, when the user inputs 70 are engaged, an electrical circuit to the coupling mechanisms 90 is closed. Electrical connection 112 may include a separate set of wires that provide power to light sources 110 regardless of whether user inputs 70 are engaged or disengaged so that status information is conveyed by the color of light sources 110 while endboard 30 is attached to frame 20 in the manner described hereinabove.

With the coupling mechanisms 90 of the endboard 30 engaged with the mating coupling mechanisms 92 of frame 20 (i.e. when the endboard 30 is attached to the frame 20), pressing the user inputs 70 closes an electrical circuit to send a signal to the control circuitry 68 to unlock the electrical brake 66 and activate the powered wheel 62. With the coupling mechanisms 90 of the endboard 30 engaged with the mating coupling mechanisms 92 of frame 20 (i.e. when the endboard 30 is attached to the frame 20), releasing the user inputs 70 opens the electrical circuit to send a signal to the control circuitry 68 to deactivate the powered wheel 62 and lock the electrical brake 66.

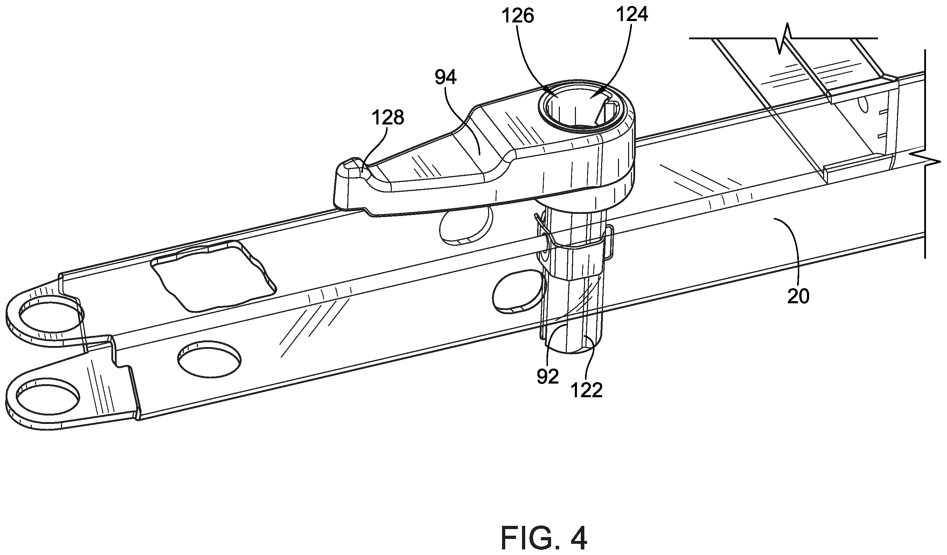

Referring to FIG. 4, the locking mechanisms 94 are provided on the frame 20 of the patient support apparatus 10. The locking mechanisms 94 are formed adjacent to the mating coupling mechanism 92 and are configured to lock the endboard 30 to the frame 20. The mating coupling mechanisms 92 each include a sleeve 122. A channel or socket 124 extends downwardly through the sleeve 122 from an opening 126. The coupling mechanisms 90 are configured to be inserted into the respective opening 126 and extended through the sleeve 122. The locking mechanism 94 is positioned around the respective sleeve 122 and is configured to rotate the sleeve 122. Each locking mechanism 94 includes a handle 128 extending outwardly from the frame 20 when in the disengaged or unlocked position. With the endboard 30 positioned on the frame 20 so that the coupling mechanism 90 is inserted into the mating coupling mechanism 92, the handle 128 is rotated by a user or caregiver to rotate the sleeve 122 and lock the coupling mechanism 90 in the mating coupling mechanism 92 to secure the endboard 30 to the frame 20.

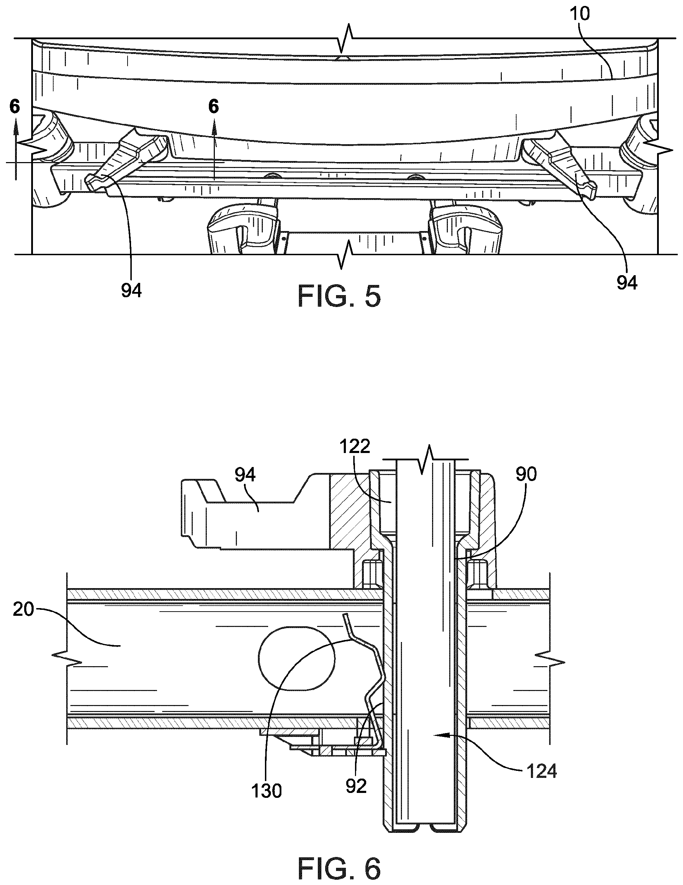

Referring more specifically to FIGs. 5 and 6, the locking mechanisms 94 are illustrated in the respective disengaged or unlocked positions with a coupling mechanism 90 inserted into a mating coupling mechanism 92. In the disengaged position, the handle 128 of the respective locking mechanism 94 is rotated to extend outwardly from the frame 20. The coupling mechanism 90 is inserted into the channel 124 of the sleeve 122. The locking mechanism 94 includes an electrical connection 130 that is positioned against the sleeve 122 when the locking mechanism 94 is in the disengaged position. In an exemplary embodiment, the electrical connection 130 is a spring blade which comprises a conductive, flexible finger. The electrical connection 130 is wired to the control circuitry 68 that controls the powered wheel 62 and the electrical brake 66.

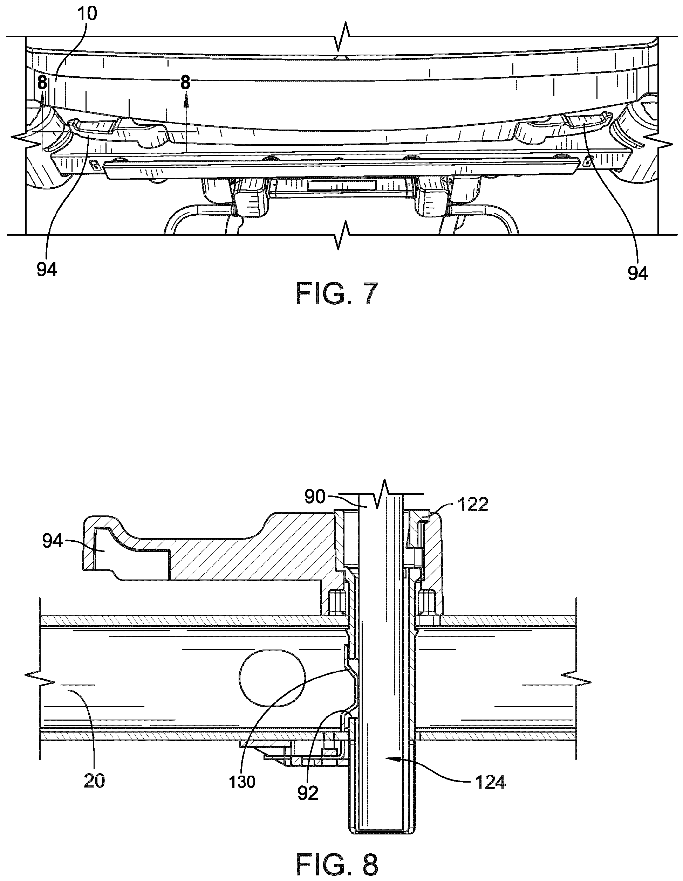

Referring to FIGs. 7 and 8, the locking mechanisms 94 are illustrated in the respective engaged positions, wherein the handle 128 of the respective locking mechanism 94 is rotated to lock the endboard 30 to the frame 20. In the engaged position, the handle 128 is rotated toward the patient support apparatus 10 so as to be generally parallel with a laterally extending end frame member of frame 20 and with bottom 82 of endboard 30. In some embodiments, the handle 128 is rotated into and stowed in a notch of the endboard 30 of patient support apparatus 10. Rotating the locking mechanisms 94 rotates the corresponding sleeve 122 so that a notch 140 formed in the sleeve 122 is rotated into a position to allow the electrical connection 130 to resiliently flex and extend through the notch 140. In this position, the electrical connection 130 engages the coupling mechanism 90 (e.g., post 90) to provide an electrical circuit between the user input 70 and the control circuitry 68. That is, with the locking mechanisms 94 in the engaged positions, an electrical circuit extends from the user inputs 70, though the coupling mechanisms 90, to the mating coupling mechanisms 92 (via the engagement between the electrical connection 130 and the coupling mechanism 90), and to the control circuitry 68. This electrical circuit is closed by depressing the user inputs 70 to unlock the electrical brake 66 and activate the powered wheel 62. This electrical circuit is opened by releasing either or both of the user inputs 70 to deactivate the powered wheel 62 and lock the electrical brake 66.

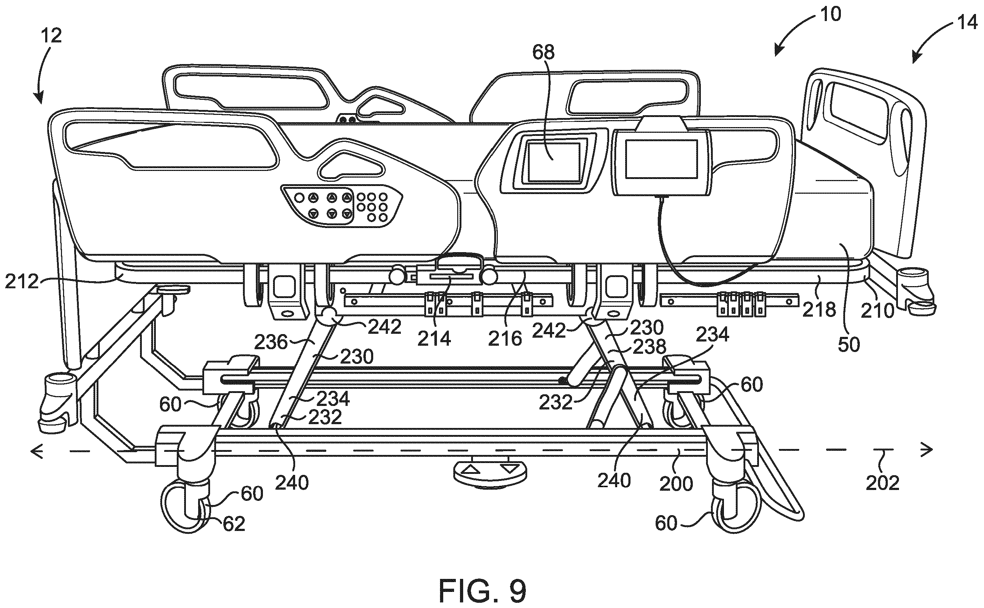

Referring to FIG. 9, the patient support apparatus 10 includes a base frame 200 having a longitudinal axis 202 extending between the head end 12 and the foot end 14. The plurality of casters 60 is coupled to the base frame 200 and includes at least one powered wheel 62 controlled by an electronic unit (described below). An articulating deck 210 is positioned above the base frame 200 and is configured to carry the mattress 50. The articulating deck 210 includes a head section 212, a seat section 214, and a thigh section 216. The head section 212 is configured to articulate relative to the seat section 214. Likewise, the thigh section 216 is configured to articulate relative to the seat section 214. In some embodiments, a foot section 218 is pivotably coupled to the foot end 14 of the thigh section 216. The foot section 218 extends and retracts to change its length, in some embodiments.

A lift assembly 230 couples the base frame 200 to an upper frame 201, which supports the articulating deck 210. The lift assembly 230 is configured to move the upper frame 201 and the articulating deck 210 relative to the base frame 200. In an exemplary embodiment, the lift assembly 230 is operable to raise, lower, and tilt the upper frame 201 relative to the base frame 200. That is, the lift assembly 230 moves the upper frame 201 and articulating deck 210 up and down relative to the base frame 200. The lift assembly 230 includes a plurality of linkages 232 configured to move the articulating deck 210 relative to the base frame 200. The plurality of linkages 232 includes at least one lift arm 234 configured to raise and lower the articulating deck 210 relative to the base frame 200. In the illustrated embodiment, the at least one lift arm 234 includes a head end pair of lift arms 236 and a foot end pair of lift arms 238. The head end pair of lift arms 236 are laterally spaced in a direction substantially perpendicular to the longitudinal axis 202 to define a space therebetween. The foot end pair of lift arms 238 are also laterally spaced in a direction substantially perpendicular to the longitudinal axis 202 to define another space therebetween. The head end pair of lift arms 236 and the foot end pair of lift arms 238 are each pivoted in unison to raise and lower at least a portion of the upper frame 201 relative to the base frame 200. In an exemplary embodiment, a lower end 240 of each lift arm 234 moves longitudinally along the base frame 200 as the articulating deck 210 is raised and lowered by the lift arms 234. That is, the lower end 240 of each lift arm 234 translates along the base frame 200 as the articulating deck 210 is raised and lowered by the lift arms 234. An upper end 242 of each lift arm 234 rotates relative to the upper frame 201 as the upper frame 201 is raised and lowered by the lift arms 234.

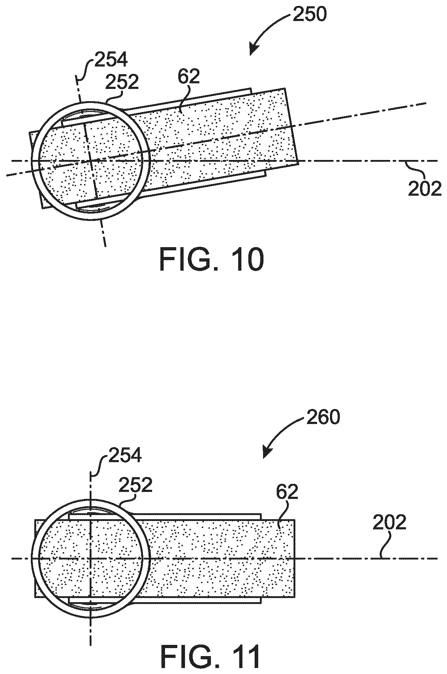

Referring to FIG. 10, the powered wheel 62 is free to swivel into any number of rotated positions 250 by rotating about a post 252 that couples the powered wheel 62 to the base frame 200. In any of the rotated positions, a wheel axis 254 of the powered wheel 62, about which the powered wheel 62 rotates, is positioned parallel to or at a non-orthogonal angle to the longitudinal axis 202. In a rotated position 250, engagement of the user input 70 by the user is ineffective to activate the at least one powered wheel 62. Accordingly, the powered wheel 62 is required to be moved to a steering position 260, as shown in FIG. 11, to activate the powered wheel 62. In some embodiments the powered wheel 62 is locked in the steering position 260 to enable activation of the powered wheel 62 by manually engaging the user input 70. In the steering position 260, the wheel axis 254 of the powered wheel 62 is positioned substantially perpendicular to the longitudinal axis 202. In an embodiment having multiple powered wheels 62, each powered wheel 62 is positioned in the steering position 260 to enable activation of the powered wheels 62 by manually engaging the user input 70.

Referring to FIG. 12, the patient support apparatus 10 includes the control circuit 68 and a plurality of actuators 270 shown in dashed lines. The plurality of actuators 270 includes lift actuators 272 that each are operable to move a pair of the lift arms 234. Each lift actuator 272 is coupled to the upper end 242 of the respective lift arm 234. For example, a foot end lift actuator 274 is operable to move the foot end pair of lift arms 238 and a head end lift actuator 276 operable to move the head end pair of lift arms 236. The plurality of actuators 270 also includes a plurality of deck actuators 280 that are operable to move at least one deck section of the articulating deck 210. For example, at least one of the deck actuators 280 is configured to move the head section 212 of the articulating deck 210. That is, the deck actuator 280 rotates the head section 212 relative to the seat section 214. Another one of the deck actuators 280 moves the thigh section 216 of the articulating deck 210 relative to the seat section 214. That is, the deck actuator 280 rotates the thigh section 216 relative to the seat section 214. The control circuit 68 is electrically coupled to a user interface 268 to receive signals from the user interface 268 that activate the lift actuators 270 and the plurality of deck actuators 280. In some embodiments, the thigh section 216 is coupled to the foot section 218 at the foot end 14 and, in some embodiments, is extendable and retractable via operation of a foot extension actuator (not shown).

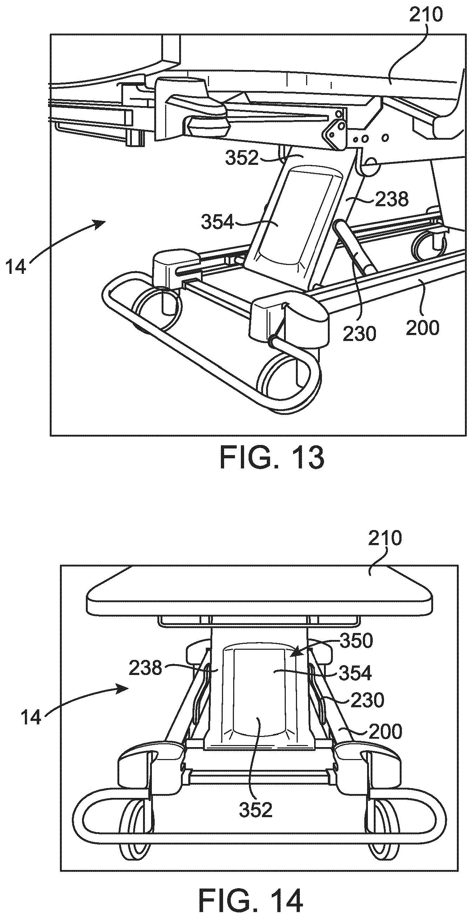

Electronic units 352 are also illustrated in dashed lines in FIG. 12. Referring to FIGs. 13 and 14, the lift assembly 230 positioned at the foot end 14 of the patient support apparatus 10 includes the foot end pair of lift arms 238. The foot end pair of lift arms 238 are spaced laterally relative to the longitudinal axis 202 to define a space 350 therebetween. A foot end electronics unit 358 is positioned in the space 350 and includes a cover panel 354 that is removable to access components within the electronics unit 352. The electronics unit 352 is carried by and moves in conjunction with the lift assembly 230. That is, the electronics unit 352 is coupled to and configured to move with the foot end pair of lift arms 238 when the articulating deck 210 is moved relative to the base frame 200. A head end electronics unit 356 (shown in FIG. 12) is also coupled to and moves with the head end pair of lift arms 236, in some embodiments. It will be appreciated that the description of the foot end electronics unit 358 herein equally applies to the head end electronics unit 356 if present. In some embodiments, therefore, a foot end electronics unit 358 is coupled to and moves with the foot end pair of lift arms 238 and the head end electronics unit 356 is coupled to and moves with the head end pair of lift arms 236.

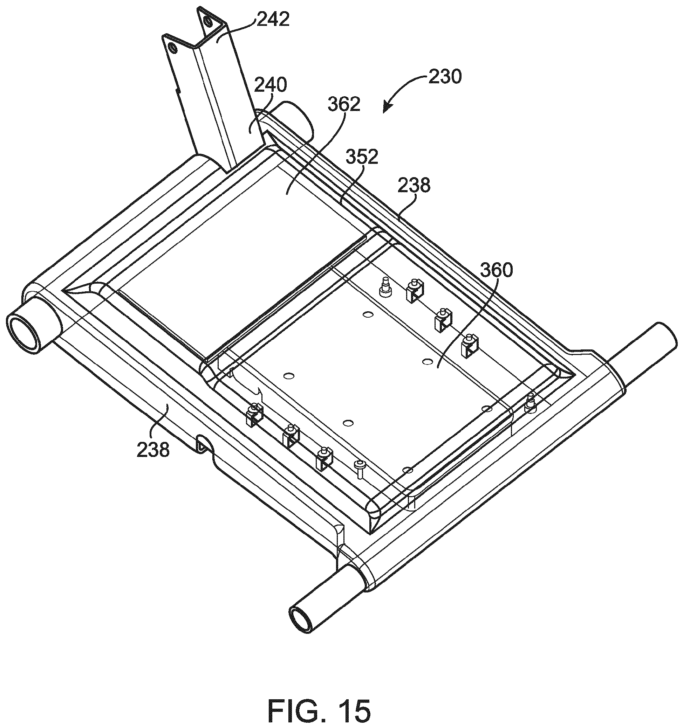

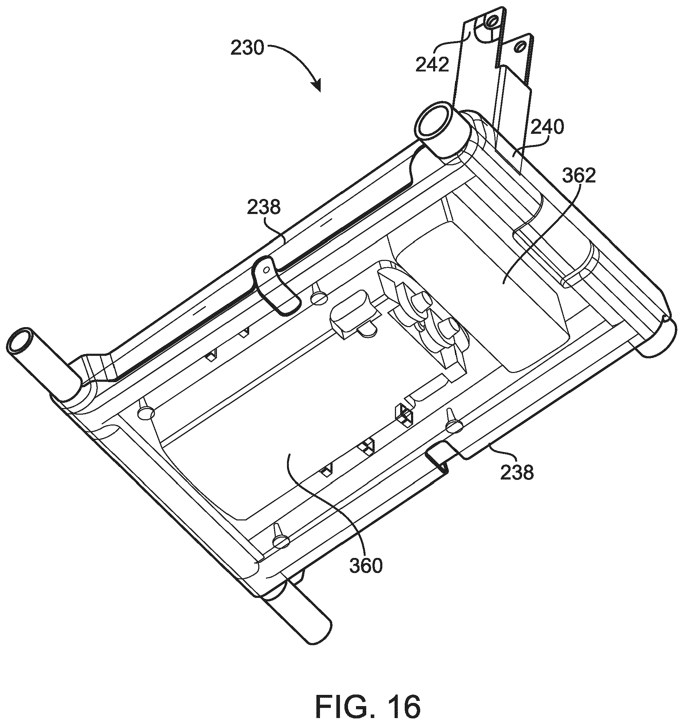

Referring to FIG. 15, the cover panel 354 is illustrated as transparent to show that the electronic unit 352 includes a battery 360 and a motor controller 362. FIG. 16 illustrates another view of the electronics unit 352 including the battery 360 and the motor controller 362 between the lift arms 238. In an exemplary embodiment, the battery 360 is electrically coupled to each of the at least one powered wheel 62, the actuators 270, and the control circuitry 68. In an exemplary embodiment, the motor controller 362 receives input signals from control circuitry 68 regarding which of the actuators 270 and powered wheel 62 to operate and directs power from the battery 360 to the selected devices 270, 62. Thus, motor controller 362 signals operation of the motor 64 to drive the at least one powered wheel 62 and also signals operation of actuators 270 as needed. In an exemplary embodiment, an electrical connection between the electronic unit 352 and the powered wheel 62, the actuators 270, and the control circuitry 68 includes conductors, such as cables and/or wires that extend along or through the base frame 200, upper frame 201, and lift assemblies 230.

In some embodiments, the foot end electronic unit 352 coupled to the foot end pair of lift arms 238 and the head end electronic unit 356 coupled to the head end pair of lift arms 236 each include a battery 360. In some embodiments, the foot end electronic unit 352 and the head end electronic unit 356 each include a motor controller 362. In some embodiments, the foot end electronic unit 352 and the head end electronic unit 356 each include a battery 360 and a motor controller 362 (as shown). In some embodiments, the foot end electronic unit 352 is electrically coupled to the foot end lift actuator 274 and the head end electronic unit 356 is electrically coupled to the head end lift actuator 276.

The electronic unit 352 is carried by and moves in conjunction with the lift assembly 230. That is, the electronic unit 352 is carried by the pair of lift arms 234 and situated within the space 350 defined between the pair of lift arms 234. The electronic unit 352 is configured to move with the pair of lift arms 234 when the upper frame 201 is moved relative to the base frame 200. In the exemplary embodiment, the electronic unit 352 is accessible when the articulating deck 210 is in a raised position to allow at least one of mounting or changing of the electronic unit 352. Moreover, the electronic unit 352 is accessible with a patient positioned on the mattress 50.

Embodiments of the invention can be described with reference to the following numbered clauses, with preferred features laid out in the dependent clauses:
1. A patient support apparatus comprising:
   a frame;
   an electrical system coupled to the frame and operable to operate an electrical function of the patient support apparatus;
   an endboard removably coupled to the frame by a coupling mechanism that provides an electrical connection to a mating coupling mechanism of the frame, the endboard having a user input in electrical communication with the coupling mechanism so that an input received from a user at the user input is communicated electrically to the electrical system via the electrical connection to operate the electrical system to operate an electrical function of the patient support apparatus.
2. The patient support apparatus of clause 1, wherein the electrical system includes a propulsion system to propel the patient support apparatus along a floor.
3. The patient support apparatus of either clause 1 or clause 2, wherein the electrical system includes a powered wheel configured to move the patient support apparatus, wherein the powered wheel is activated when the input is received from the user at the user input.
4. The patient support apparatus of any preceding clause, wherein the electrical system includes an electrical brake configured to prevent movement of the patient support apparatus, wherein the electrical brake is activated when the user input is released by the user.
5. The patient support apparatus of clause 4, wherein the user input includes a plurality of activation states, wherein the powered wheel moves the patient support apparatus in a first direction, when the user input is in a first activation state, and wherein the powered wheel moves the patient support apparatus in a second direction, when the user input is in a second activation state.
6. The patient support apparatus of either clause 4 or clause, wherein the user input includes a plurality of activation states, wherein the electrical brake is released and the powered wheel is placed in idle, when the user input is in an idle activation state, and wherein the powered wheel begins moving the patient support apparatus, when the user input is in a movement activation state.
7. The patient support apparatus of any preceding clause, wherein the coupling mechanism includes a post configured to be inserted into the mating coupling mechanism.
8. The patient support apparatus of any preceding clause, wherein the mating coupling mechanism includes a post configured to be inserted into the coupling mechanism.
9. The patient support apparatus of any preceding clause, wherein the endboard is a footboard.
10. The patient support apparatus of any preceding clause, wherein the endboard is a headboard.
11. The patient support apparatus of any preceding clause, further comprising a locking mechanism configured to secure the endboard to the frame.
12. The patient support apparatus of clause 11, wherein the locking mechanism is moveable between a first position and a second position to provide the electrical connection between the coupling mechanism and the mating coupling mechanism.
13. The patient support apparatus of either clause 11 or clause12, wherein the locking mechanism includes a spring blade to provide the electrical connection between the coupling mechanism and the mating coupling mechanism.
14. The patient support apparatus of any preceding clause, wherein:
   the user input is a first user input, and
   the endboard includes a second user input, wherein the first user input is positioned on a first side of the endboard, and the second user input is positioned on a second side of the endboard.
15. The patient support apparatus of clause 14, wherein both the first user input and the second user input are required to be engaged to control the electrical system of the patient support apparatus.
16. The patient support apparatus of any preceding clause, further comprising a light source to illuminate the user input.
17. The patient support apparatus of clause 16, wherein the light source illuminates the user input in a color that indicates that the electrical system is capable of activation.
18. The patient support apparatus of either clause 16 or clause 17, wherein the light source illuminates the user input in a color that indicates that the electrical system requires charging.
19. The patient support apparatus of any one of clauses 16 to 18, wherein the light source illuminates the user input in a color that indicates that the electrical system is charging.
20. The patient support apparatus of any one of clauses 16 to 19, wherein a brightness of the light source indicates a charging level of the electrical system.
21. A patient support apparatus comprising:
   a frame,
   an endboard configured to couple to the frame, the endboard having a user input configured to control an electrical system of the patient support apparatus,
   a post extending from the endboard,
   a channel formed in a sleeve positioned in the frame, the sleeve having a notch formed therein, wherein the endboard is coupled to the frame by inserting the post into the channel in the sleeve, and
   an electrical connection aligned with the sleeve, wherein the electrical connection is configured to extend through the notch formed in the sleeve to engage the post when the endboard is coupled to the frame so that an input received from a user at the user input is communicated electrically to the electrical system to operate the electrical system to operate an electrical function of the patient support apparatus.
22. The patient support apparatus of clause 21, wherein the electrical system includes a powered wheel configured to move the patient support apparatus, wherein the powered wheel is activated when the input is received from the user at the user input.
23. The patient support apparatus of either clause 21 or clause 22, wherein the electrical system includes an electrical brake configured to prevent movement of the patient support apparatus, wherein the electrical brake is activated when the user input is released by the user.
24. The patient support apparatus of clause 23, wherein the user input includes a plurality of activation states, wherein the powered wheel moves the patient support apparatus in a forward direction, when the user input is in a first activation state, and wherein the powered wheel moves the patient support apparatus in a backward direction, when the user input is in a second activation state.
25. The patient support apparatus of either clause 23 or clause 24, wherein the user input includes a plurality of activation states, wherein the electrical brake is released and the powered wheel is placed in idle, when the user input is in an idle activation state, and wherein the powered wheel begins moving the patient support apparatus, when the user input is in a movement activation state.
26. The patient support apparatus of any one of clauses 21 to 25, wherein the endboard is a footboard.
27. The patient support apparatus of any one of clauses 21 to 26, wherein the endboard is a headboard.
28. The patient support apparatus of any one of clauses 21 to 27, further comprising a locking mechanism configured to secure the endboard to the frame.
29. The patient support apparatus of clause 28, wherein the locking mechanism rotates the sleeve so that the electrical connection extends though the notch.
30. The patient support apparatus of any one of clauses 21 to 29, wherein:
   the user input is a first user input, and
   the patient support apparatus includes a second user input, wherein the first user input is positioned on a first side of the endboard, and the second user input is positioned on a second side of the endboard.
31. The patient support apparatus of clause 30, wherein both the first user input and the second user input are required to be engaged to control the electrical system.
32. The patient support apparatus of any one of clauses 21 to 31, further comprising a light source to illuminate the user input.
33. The patient support apparatus of clause 32, wherein the light source illuminates the user input in a color that indicates that the electrical system is capable of activation.
34. The patient support apparatus of either clause 32 or clause 33, wherein the light source illuminates the user input in a color that indicates that the electrical system requires charging.
35. The patient support apparatus any one of clauses 32 to 34, wherein the light source illuminates the user input in a color that indicates that the electrical system is charging.
36. The patient support apparatus of any one of clauses 32 to 35, wherein a brightness of the light source indicates a charging level of the electrical system.
37. A patient support apparatus comprising:
   a frame,
   an endboard configured to couple to the frame, the endboard having a user input configured to control an electrical system of the patient support apparatus,
   a channel formed in a sleeve positioned in the endboard, the sleeve having a notch formed therein,
   a post extending from the frame, wherein the endboard is coupled to the frame by inserting the post into the channel in the sleeve, and
   an electrical connection aligned with the sleeve, wherein the electrical connection is configured to extend through the notch formed in the sleeve to engage the post when the endboard is coupled to the frame so that an input received from a user at the user input is communicated electrically to the electrical system to operate the electrical system to operate an electrical function of the patient support apparatus.
38. A patient support apparatus comprising:
   a base frame having a longitudinal axis extending between a head end and a foot end, a plurality of casters coupled to the base frame, wherein the plurality of casters includes at least one powered wheel,
   an articulating deck positioned above the base frame and configured to carry a patient support surface,
   a pair of lift arms coupling the base frame to the articulating deck, the pair of lift arms being laterally spaced in a direction substantially perpendicular to the longitudinal axis to define a space therebetween, the pair of lift arms configured to move the articulating deck relative to the base frame, and
   an electronic unit electrically coupled to the at least one powered wheel, wherein the electronic unit is carried by the pair of lift arms and situated within the space defined between the pair of lift arms.
39. The apparatus of clause 38, wherein the pair of lift arms are pivoted in unison to raise and lower a portion of the articulating deck relative to the base frame.
40. The apparatus of either clause 38 or 39, wherein the electronic unit includes a battery for powering the at least one powered wheel and/or a motor controller to signal operation of the at least one powered wheel.
41. The apparatus of any one of clauses 38 to 40, wherein lower ends of the pair of lift arms move longitudinally along the base frame as the articulating deck is moved by the pair of lift arms.
42. The apparatus of any one of clauses 38 to 41, wherein the electronic unit is configured to move with the pair of lift arms when the articulating deck is moved relative to the base frame.
43. The apparatus of any one of clauses 38 to 42, wherein the at least one powered wheel is activated with a user input that is engaged manually by a user.
44. The apparatus of clause 43, wherein engagement of the user input by the user is ineffective to activate the at least one powered wheel until the at least one powered wheel is locked into a steering position.
45. The apparatus of clause 44, wherein, in the steering position, the at least one powered wheel is positioned to rotate about a wheel axis that is substantially perpendicular to the longitudinal axis.
46. The apparatus of any one of clauses 38 to 45, further comprising a lift actuator that is operable to move the pair of lift arms and wherein the electronic unit is also electrically coupled to the actuator.
47. The apparatus of clause 46, further comprising at least one deck actuator that is operable to move at least one deck section of the articulating deck and wherein the electronic unit is also electrically coupled to the deck actuator.
48. The apparatus of any one of clauses 38 to 47, wherein an electrical connection between the electronic unit and the at least one powered wheel extends through the base frame.
49. The apparatus of any one of clauses 38 to 48, wherein the electronic unit is accessible when the articulating deck is in a raised position to allow at least one of mounting or changing of the electronic unit.
50. The apparatus of clause 49, wherein the electronic unit is accessible with a patient positioned on the patient support surface.
51. The apparatus of any one of clauses 38 to 50, wherein the pair of lift arms includes at least one of a head end pair of lift arms or a foot end pair of lift arms.
52. The apparatus of any one of clauses 38 to 51, wherein the pair of lift arms includes at least one of a head end pair of lift arms and a foot end pair of lift arms, and wherein the electronic unit comprises a first electronic unit coupled to the head end pair of lift arms and a second electronic unit coupled to the foot end pair of lift arms.
53. The apparatus of clause 52, wherein the first and second electronic units each comprises a battery.
54. The apparatus of either clause 52 or clause 53, wherein the first and second electronic units each comprises a motor controller.
55. The apparatus of any one of clauses 52 to 54, wherein the first and second electronic units each comprises a battery and a motor controller.
56. The apparatus of any one of clauses 52 to 55, further comprising a first lift actuator operable to move the head end pair of lift arms and a second lift actuator operable to move the foot end pair of lift arms, and wherein the first electronic unit is electrically coupled to the first lift actuator and the second electronic unit is electrically coupled to the second lift actuator.
57. A patient support apparatus comprising:
   a base frame having at least one powered wheel coupled thereto,
   an articulating deck positioned above the base frame and configured to carry a patient support surface,
   a lift assembly coupling the base frame to the articulating deck and configured to move the articulating deck relative to the base frame, and
   an electronic unit electrically coupled to the at least one powered wheel, wherein the electronic unit is carried by and moves in conjunction with the lift assembly.
58. The apparatus of clause 57, wherein the lift assembly raises and lowers a portion of the articulating deck relative to the base frame.
59. The apparatus of either clause 57 or clause 58, wherein the electronic unit includes a battery for powering the at least one powered wheel and/or a motor controller to signal operation of the at least one powered wheel.
60. The apparatus of any one of clauses 57 to 59, wherein a lower end of the lift assembly moves longitudinally along the base frame as the articulating deck is moved by the lift assembly.
61. The apparatus of any one of clauses 57 to 60, wherein the at least one powered wheel is activated with a user input that is engaged manually by a user.
62. The apparatus of clause 61, wherein engagement of the user input by the user is ineffective to activate the at least one powered wheel until the at least one powered wheel is locked into a steering position.
63. The apparatus of clause 62, wherein, in the steering position, the at least one powered wheel is positioned to rotate about a wheel axis that is substantially perpendicular to the longitudinal axis.
64. The apparatus of any one of clauses 57 to 63, further comprising a lift actuator that is operable to move the lift assembly and wherein the electronic unit is also electrically coupled to the actuator.
65. The apparatus of clause 64, further comprising at least one deck actuator that is operable to move at least one deck section of the articulating deck and wherein the electronic unit is also electrically coupled to the deck actuator.
66. The apparatus of any one of clauses 57 to 65, wherein an electrical connection between the electronic unit and the at least one powered wheel extends through the base frame.
67. The apparatus of any one of clauses 57 to 66, wherein the electronic unit is accessible when the articulating deck is in a raised position to allow at least one of mounting or changing of the electronic unit.
68. The apparatus of any one of clauses 57 to 67, wherein the electronic unit is accessible with a patient positioned on the patient support surface.
69. The apparatus of any one of clauses 57 to 68, wherein the lift assembly includes at least one of a head end lift assembly or a foot end lift assembly.
70. The apparatus of any one of clauses 57 to 69, wherein the lift assembly includes at least one of a head end lift assembly and a foot end lift assembly, and wherein the electronic unit comprises a first electronic unit coupled to the head end lift assembly and a second electronic unit coupled to the foot end lift assembly.
71. The apparatus of any one of clauses 57 to 70, wherein the first and second electronic units each comprises a battery.
72. The apparatus of any one of clauses 57 to 71, wherein the first and second electronic units each comprises a motor controller.
73. The apparatus of any one of clauses 57 to 72, wherein the first and second electronic units each comprises a battery and a motor controller.
74. The apparatus of any one of clauses 57 to 73, further comprising a first lift actuator operable to move the head end lift assembly and a second lift actuator operable to move the foot end lift assembly, and wherein the first electronic unit is electrically coupled to the first lift actuator and the second electronic unit is electrically coupled to the second lift actuator.
75. A patient support apparatus comprising:
   a base frame having at least one powered wheel coupled thereto, wherein the at least one powered wheel is controlled by a control circuit,
   an articulating deck positioned above the base frame and configured to carry a patient support surface,
   a plurality of linkages configured to move the articulating deck relative to the base frame, wherein the plurality of linkages includes a lift arm configured to raise and lower the articulating deck relative to the base frame,
   an electronic unit electrically coupled to each of the at least one powered wheel, the linkages, and the control circuit, wherein the electronic unit is carried by the lift arm.
76. The apparatus of clause 75, wherein the lift arm raises and lowers a portion of the articulating deck relative to the base frame.
77. The apparatus of clause 75, wherein the electronic unit includes a battery for powering the at least one powered wheel and/or a motor controller to signal operation of the at least one powered wheel.
78. The apparatus of clause 75, wherein a lower end of the lift arm moves longitudinally along the base frame as the articulating deck is moved by the lift arm.
79. The apparatus of clause 75, wherein the electronic unit is configured to move with the lift arm when the articulating deck is moved relative to the base frame.
80. The apparatus of clause 75, wherein the at least one powered wheel is activated with a user input that is engaged manually by a user.
81. The apparatus of clause 80, wherein engagement of the user input by the user is ineffective to activate the at least one powered wheel until the at least one powered wheel is locked into a steering position.
82. The apparatus of clause 81, wherein, in the steering position, the at least one powered wheel is positioned to rotate about a wheel axis that is substantially perpendicular to the longitudinal axis.
83. The apparatus of any one of clauses 75 to 82, wherein an electrical connection between the electronic unit and the at least one powered wheel extends through the base frame.
84. The apparatus of any one of clauses 75 to 83, wherein the electronic unit is accessible when the articulating deck is in a raised position to allow at least one of mounting or changing of the electronic unit.
85. The apparatus of clause 84, wherein the electronic unit is accessible with a patient positioned on the patient support surface.
86. The apparatus of any one of clauses 77 to 85, wherein the lift arm includes at least one of a head end lift arm or a foot end lift arm.
87. The apparatus of any one of clauses 75 to 86, wherein the lift arm includes at least one of a head end lift arm and a foot end lift arm, and wherein the electronic unit comprises a first electronic unit coupled to the head end lift arm and a second electronic unit coupled to the foot end lift arm.
88. The apparatus of clause 87, wherein the first and second electronic units each comprises a battery.
89. The apparatus of clause 87, wherein the first and second electronic units each comprises a motor controller.
90. The apparatus of clause 87, wherein the first and second electronic units each comprises a battery and a motor controller.
91. The apparatus of clause 87, further comprising a first lift actuator operable to move the head end lift arm and a second lift actuator operable to move the foot end lift arm, and wherein the first electronic unit is electrically coupled to the first lift actuator and the second electronic unit is electrically coupled to the second lift actuator.

## Claims

1. A patient support apparatus comprising:
a frame;
an electrical system coupled to the frame and operable to operate an electrical function of the patient support apparatus;
an endboard removably coupled to the frame by a coupling mechanism that provides an electrical connection to a mating coupling mechanism of the frame, the endboard having a user input in electrical communication with the coupling mechanism so that an input received from a user at the user input is communicated electrically to the electrical system via the electrical connection to operate an electrical function of the patient support apparatus; and
a locking mechanism configured to secure the endboard to the frame, wherein the locking mechanism is moveable between a first position and a second position to provide the electrical connection between the coupling mechanism and the mating coupling mechanism.

2. The patient support apparatus of claim 1, wherein the electrical system includes a propulsion system to propel the patient support apparatus along a floor.

3. The patient support apparatus of either claim 1 or claim 2, wherein the electrical system includes a powered wheel configured to move the patient support apparatus, wherein the powered wheel is activated when the input is received from the user at the user input.

4. The patient support apparatus of claim 3, further comprising an electronic unit electrically coupled to the powered wheel, wherein the electronic unit is carried by a pair of lift arms and situated within the space defined between the pair of lift arms.

5. The patient support apparatus of claim 3, wherein the electrical system includes an electrical brake configured to prevent movement of the patient support apparatus, wherein the electrical brake is activated when the user input is released by the user.

6. The patient support apparatus of claim 5, wherein the user input includes a plurality of activation states, wherein the powered wheel moves the patient support apparatus in a first direction, when the user input is in a first activation state, and wherein the powered wheel moves the patient support apparatus in a second direction, when the user input is in a second activation state.

7. The patient support apparatus of claim 5, wherein the user input includes a plurality of activation states, wherein the electrical brake is released and the powered wheel is placed in idle, when the user input is in an idle activation state, and wherein the powered wheel begins moving the patient support apparatus, when the user input is in a movement activation state.

8. The patient support apparatus of any preceding claim, wherein the coupling mechanism includes a post configured to be inserted into the mating coupling mechanism.

9. The patient support apparatus of any preceding claim, wherein the mating coupling mechanism includes a post configured to be inserted into the coupling mechanism.

10. The patient support apparatus of any preceding claim, wherein the endboard is a footboard.

11. The patient support apparatus of any preceding claim, wherein the endboard is a headboard.

12. The patient support apparatus of any preceding claim, wherein the locking mechanism includes a spring blade to provide the electrical connection between the coupling mechanism and the mating coupling mechanism.

13. The patient support apparatus of any preceding claim, wherein:
the user input is a first user input, and
the endboard includes a second user input, wherein the first user input is positioned on a first side of the endboard, and the second user input is positioned on a second side of the endboard.

14. The patient support apparatus of any preceding claim , further comprising a light source to illuminate the user input.

15. The patient support apparatus of claim 14, wherein the light source illuminates the user input in a color that indicates that the electrical system is at least one of capable of activation, requires charging, or is charging.
